# FASCICULE DE BREVET EUROPEEN

(11) **EP 0 955 314 B1**
(45) Date de publication et mention de la délivrance du brevet: **03.12.2003**
(21) Numéro de dépôt: 99400613.8
(22) Date de dépôt: 12.03.1999
(51) Int. Cl.: C07K 14/605, A61K 38/30

(54) **Composés peptidiques analogues du glucagon-like-peptide-1 (7-37), leur procédé de préparation et les compositions pharmaceutiques qui les contiennent**
Peptidanaloge des Glucagon-ähnlichen-Peptids-1 (7-37), deren Herstellung und pharmazeutische Zusammensetzungen
Peptide analogues of glucagon-like-peptide-1 (7-37), their preparation and pharmaceutical compositions

(30) Priorité: 10.04.1998 FR 9804559
(43) Date de publication de la demande: 10.11.1999
(73) Titulaire: LES LABORATOIRES SERVIER, 92200 Neuilly sur Seine (FR)
(72) Inventeur: Calas, Bernard, 34000 Montpellier (FR); Grassy, Gérard, 34470 Perols (FR); Chavanieu, Alain, 34820 Assas (FR); Sarrauste de Menthiere, Cyril, 34000 Montpellier (FR); Renard, Pierre, 78150 Le Chesnay (FR); Pfeiffer, Bruno, 95320 Saint Leu La Foret (FR); Manechez, Dominique., 59650 Villeneuve D ascq (FR)

(56) Documents cités:
- EP-A- 0 658 568
- EP-A- 0 733 644
- WO-A-91/11457
- WO-A-93/25579
- WO-A-98/43658
- SVETLANA MOJSOV: "STRUCTURAL REQUIREMENTS FOR BIOLOGICAL ACTIVITY OF GLUCAGON-LIKE PEPTIDE-I" INTERNATIONAL JOURNAL OF PEPTIDE AND PROTEIN RESEARCH, vol. 40, no. 3 / 04, 1 septembre 1992 (1992-09-01), pages 333-343, XP000311244
- GEFEL D ET AL: "GLUCAGON-LIKE PEPTIDE-I ANALOGS: EFFECTS ON INSULIN SECRETION AND ADENOSINE 3',5'-MONOPHOSPHATE FORMATION" ENDOCRINOLOGY, vol. 126, no. 4, 1 avril 1990 (1990-04-01), pages 2164-2168, XP000574862
- SUZUKI S ET AL: "COMPARISON OF THE EFFECTS OF VARIOUS C-TERMINAL AND N-TERMINAL FRAGMENT PEPTIDES OF GLUCAGON-LIKE PEPTIDE-1 ON INSULIN AND GLUCAGON RELEASE FROM THE ISOLATED PERFUSED RAT PANCREAS" ENDOCRINOLOGY, vol. 125, no. 6, 1 décembre 1989 (1989-12-01), pages 3109-3114, XP000574778

## Description

La présente invention concerne de nouveaux composés peptidiques analogues du Glucagon-Like-Peptide-1 (7-37), leur procédé de préparation et les compositions pharmaceutiques qui les contiennent.

Les Glucagon-Like-Peptides-1 (7-37) et (7-36) NH₂ (^{t}GLP-1) sont des peptides d'origine intestinale, fortement impliqués dans le contrôle de l'homéostase glucidique. Ces peptides sont les principaux médiateurs de l'axe entéro-insulaire et agissent en se fixant à des récepteurs spécifiques.

Le ^{t}GLP-1 agit de manière prépondérante au niveau pancréatique en exerçant un effet puissant de stimulation de l'insulino-sécrétion par les cellules β, de manière glucose dépendante (S Mojsov et al., J Clin Invest., 1987, 79, 619, et J J. Holst, F E B S Letters, 1987, 211. 169). Cette stimulation s'accompagne d'une stimulation de la libération de somatostatine et d'une inhibition de la libération de glucagon.

Parallèlement à ces effets pancréatiques, le ^{t}GLP-1 a pour effet de ralentir la vidange gastrique, de diminuer les sécrétions acides et de stimuler l'utilisation périphérique du glucose au niveau musculaire, hépatique, et adipocytaire (M L Villanueva et al., Diabetologia, 1994. 37, 1163, D J Drucker, Diabetes, 1998, 47, 159),

Des études récentes ont également montré que le ^{t}GLP-1 pouvait avoir une influence sur le comportement alimentaire en inhibant la prise de nourriture et de boisson, par action sur les centres de la satiété (M D Turton et al., Nature, 1996, 379, 69).

Le ¹GLP-1 possède donc de multiples applications thérapeutiques potentielles, en particulier dans le traitement du diabète de type II, non insulino-dépendant, de l'obésité, et dans le diabète de type I.

Toutefois, comme beaucoup de peptides hormonaux, il possède une demi-vie plasmatique assez courte, inférieure à 2 minutes (T.J Kieffer et al., Endocrinology 1995, 136, 3585), ce qui limite son utilisation.

L'utilisation du peptide naturel GLP₁ (7-37) pour ses propriétés insulinotropiques a été largement décrite, que ce soit pour le peptide naturel GLP₁ (7-37) ou GLP₁ (7-36) NH₂ seul, sous forme de sels, esters ou amides (US 5616492, WO 8706941, WO 9011296), associé à des phospholipides (WO 9318785) ou associé à d'autres substances hypoglycémiantes (WO 9318786). Des analogues modifiés en quelques positions de la séquence naturelle ont également été étudiés (EP 733644, EP 708179. EP 658568. WO 9111457) dans le but de concevoir des dérivés aussi puissants que le GLP₁ (7-37) et mieux absorbés.

Les composés de la présente invention possèdent une structure originale dérivant de celle du ^{t}GLP-1 par modifications de plusieurs résidus et par suppression de l'arginine en position 36. Outre le fait qu'ils soient nouveaux, ces composés possèdent des propriétés pharmacologiques intéressantes, dues à leur caractère agoniste pour les récepteurs au ^{t}GLP-1. Les modifications apportées présentent de plus l'avantage d'augmenter considérablement la stabilité métabolique des composés de l'invention et leurs conférent ainsi une durée d'action supérieure au peptide naturel. Ces propriétés rendent ces dérivés particulièrement intéressant pour le traitement des pathologies pour lesquelles le ^{t}GLP-1 intervient, notamment dans le traitement du diabète de type Il non msulino-dépendant, de l'obésité, et dans le diabète de type 1.

La présente invention concerne les composés peptidiques suivants :

•  His - Ala - Glu - Gly - Thr - Phe - Thr - Ser - Asp - Val - Ser - Ser - Tyr - (D)-Leu -
Glu - Gly - Gln - Ala - Ala - Lys - Glu - Phe - Ile - Ala - Trp - Leu - Val - Lys - Gly -
Arg - Gly - NH2

• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2

• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

• His - Ala - Gln - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2

• His - Ala - His - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2

His - Ala - (D)-Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2

• His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2

• His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Leu- Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Val - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

Afp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

•  Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - NH2

• Phe - (D)-Ala - Gln - Gly - Thr - Phe - Thr - Ser - Asp - Val - Ser - Ser - Val - (D)-Leu -
Glu - Gly - Gln - Ala - Ala - Lys - Glu - Phe - Ile - Ala - Trp - Leu - Val - Lys - Gly -
Gly - NH2

His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Ile - Ile - Ala - Trp - Leu - Val - Lys - Gly - Gly - NH2

•  His - Ala - Glu - Gly - Thr - Phe - Thr - Ser - Asp - Val - Ser - Ser - Tyr - Leu - Glu -
Gly - Gln - Ala - Ala - Lys - Glu - Val - Ile - Ala - Trp - Leu - Val - Lys - Gly - Gly -
NH2

His - Ala - Glu - Gly - Thr - Phe - Thr - Ser - Asp - Val - Ser - Ser - Tyr - Leu - Glu -
Gly - Gln - Ala - Ala - Lys - Glu - His - Ile - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Asp - Ile - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Phe - Leu - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Phe - Val - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Phe - Tyr - Ala - Trp - Leu - Val - Lys - Gly - Gly -
NH2

•  His - Ala - Glu - Gly - Thr - Phe - Thr - Ser - Asp - Val - Ser - Ser - Tyr - Leu - Glu -
Gly - Gln - Ala - Ala - Lys - Glu - Phe - Arg - Ala - Trp - Leu - Val - Lys - Gly - Gly -
NH2

• His - Ala - Glu - Gly - Thr - Phe - Thr - Ser - Asp - Val - Ser - Ser - Tyr - Leu - Glu -
Gly - Gln - Ala - Ala - Lys - Glu - Phe - Glu - Ala - Trp - Leu - Val - Lys - Gly - Gly -
NH2

• Phe - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

• His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg-
Gly - NH2

• His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

• His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

• His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

•  His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH₂

L'invention s'étend également au procédé de préparation desdites composés peptidiques qui peuvent être obtenus par différentes méthodes telles que la synthèse séquentielle sur phase solide. la synthèse et le couplage de fragments en solution, la synthèse enzymatique, ou en utilisant des techniques de biologie moléculaire.

Les méthodes générales de synthèse des peptides sur phase solide ont été décrites par B W Erickson et R B. Merrifield ("The proteins", Solid Phase Peptide Synthesis, 3^{ème} édition, 1976, 257).

La synthèse sur phase solide est réalisée sur un automate qui effectue de façon répétitive et programmable des cycles de déprotection, couplage et lavages nécessaires à l'introduction séquentielle des acides aminés dans la chaîne peptidique.

L'acide aminé C-terminal est fixé sur une résine conventionnellement utilisée pour la préparation de polypeptides, de préférence un polystyrène réticulé à l'aide de 0,5 à 3,0 % de divinylbenzène et muni de restes activés qui permettent la fixation covalente du premier acide aminé sur la résine Le choix approprié de la résine permet la formation après synthèse d'une fonction C-terminale acide carboxylique, amide, alcool ou ester.

Les acides aminés sont alors introduits un à un dans l'ordre déterminé par l'opérateur. Chaque cycle de synthèse correspondant à l'introduction d'un acide aminé, comporte une déprotection, N-terminale de la chaîne peptidique, des lavages successifs destinés à éhminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphénylphosphorylazide (DPPA).

Des activations par formation d'anhydrides mixtes ou symétriques sont également possibles.

Chaque acide aminé est introduit dans le réacteur 6 fois en excès environ, par rapport au degré de substitution de la résine et en quantité environ équivalent par rapport aux agents de couplage. La réaction de couplage peut être vérifiée à chaque étape de la synthèse par le test de réaction à la ninhydrine décrit par E KAISER et Coll (Anal Biochem , 34, 595, 1970).

Après assemblage de la chaîne peptidique sur la résine, un traitement approprié, par exemple à l'aide d'un acide fort tel que l'acide trifluoroacétique, ou l'acide fluorhydrique en présence d'anisole, d'éthanedithiol ou de 2-méthylindole sert à séparer le peptide de la résine ainsi qu'à libérer le peptide de ses groupes protecteurs. Le composé est alors purifié par des techniques classiques de purification, notamment chromatographiques.

Les peptides de la présente invention peuvent être également obtenus par couplage en solution de fragments peptidiques sélectivement protégés, qui peuvent être préparés eux-mêmes soit sur phase solide, soit en solution. L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide Les methodes d'activation lors des couplages sont également analogues à celles employées dans la synthèse sur phase solide.

Les peptides de la présente invention peuvent aussi être obtenus en utilisant des techniques de biologie moléculaire, en utilisant des séquences d'acide nucléique codant pour ces peptides Ces séquences peuvent être des ARN ou des ADN et être associées à des séquences de contrôle et/ou insérées dans des vecteurs. Ces derniers sont ensuite transfectés dans des cellules hôtes, par exemple des bactéries. La préparation de ces vecteurs ainsi que leur production ou expression dans un hôte sont réalisées par les techniques classiques de biologie moléculaire et de génie génétique.

La présente invention a également pour objet les compositions pharmaceutiques renfermant comme principe actif au moins un composé peptidique selon l'invention ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les suppositoires, crèmes, pommades, gels dermiques, dispositifs transdermiques, les aérosols, ampoules buvables et injectables.

La posologie varie selon l'âge et le poids du patient, la nature et la sévérité de l'affection ainsi que la voie d'administration.

Celle-ci peut être orale (incluant la voie inhalée, gingivale et sublinguale), nasale, rectale, parentérale ou transdermique. D'une manière générale, elle s'échelonne entre 10 µg et 500 mg pour un traitement en une ou plusieurs prises par 24 heures selon la voie d'administration et la forme galénique utilisée.

Par convention dans les exemples ci-dessous, les acides aminés dont les abréviations commencent par une lettre majuscule sans aucune autre précision sont de configuration L Les acides aminés de configuration D seront précédés du symbole (D).

### EXEMPLE 1 :

His - Ala - Glu - Gly - Thr - Phe - Thr - Ser - Asp - Val - Ser - Ser - Tyr - (D)-Leu -
Glu - Gly - Gln - Ala - Ala - Lys - Glu - Phe - Ile - Ala - Trp - Leu - Val - Lys - Gly -
Arg - Gly - NH2

Le composé de l'exemple 1 est synthétisé à l'échelle de 0,1 mmole à partir d'une résine Fmoc - PAL - PEG - PS, en utilisant un appareil en flux continu, en suivant le protocole répétitif suivant.

| **N° opération** | **Fonction** | **Solvant / Réactif** | **Temps** |
|---|---|---|---|
| 1 | lavage | DMF | 5 min |
| 2 | déprotection | 20 % pipéridine/DMF | 15 min |
| 3 | lavage | DMF | 15 min |
| 4 | couplage | Acide aminé / DIPCDI / HOBt | 60 min |
| 5 | lavage | 20 % pipéridine / DMF | 5 min |
| 6 | lavage | DMF | 5 min |
| 7 | lavage | dichlorométhane | 5 min |

Chaque opération, effectuée à température ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Chaque acide aminé (6 équivalents) est assemblé en utilisant la diisopropylcarbodiimide (DIPCDI) en présence d'hydroxy-1-benzotriazole (HOBt) comme agent de couplage.

Après incorporation du dernier acide aminé, on obtient un peptide, protégé sur ses chaînes latérales et fixé sur la résine. Le support est alors séché sous vide poussé pendant 3 heures Il est ensuite traité par 50 ml de réactif K (acide trifluoroacétique 82,5 %, phénol 5 %, eau 5 %, thioanisole 5 %, éthanedithiol 2,5 %). Le mélange est alors laissé à température ambiante avec agitation occasionnelle pendant 12 heures, puis filtré dans un erlen contenant environ 200 ml d'éther éthylique. Le peptide précipite. et est isolé par filtration ou centrifugation. Il est ensuite séché sous vide poussé en presence de pastilles de potasse pendant 12 heures, puis purifié par CLHP préparative sur colonne de phase inverse (C₁₈) en utilisant un gradient eau-acétonitrile. Les fractions contenant le peptide sont rassemblées puis lyophilisées.
*Spectre de masse :* ESI - MS m/z = 3356

Les exemples suivants ont été préparés en utilisant le mode opératoire décrit dans l'exemple 1, en utilisant les acides aminés nécessaires.

### EXEMPLE 2 :

Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2

*Spectre de masse* ESI - MS: m/z = 3404

### EXEMPLE 3 :

Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3404

### EXEMPLE 4 :

His - Ala - Gln - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3290

### EXEMPLE 5 :

His - Ala - His - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3299

### EXEMPLE 6 :

His - Ala - (D)-Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2

*Spectre de masse*. ESI - MS : m/z = 3184

### EXEMPLE 7 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2

*Spectre de masse :* ES1 - MS : m/z = 3188

### EXEMPLE 8 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3186

### EXEMPLE 9 :

His - Leu- Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3241

### EXEMPLE 10 :

His - Val - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3227

### EXEMPLE 11 :

Afp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

avec Afp représentant 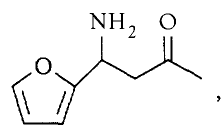 résidu correspondant à l'acide 3-amino-3-(2-furyl)propanoïque
*Spectre de masse :* ESI - MS : m/z = 3196

### EXEMPLE 12 :

His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3180

### EXEMPLE 13 :

His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3152

### EXEMPLE 14 :

His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3194

### EXEMPLE 15 :

Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - NH2

*Spectre de masse :* ESI - MS : m/z = 3274

### EXEMPLE 16 :

Phe - (D)-Ala - Gln - Gly - Thr - Phe - Thr - Ser - Asp - Val - Ser - Ser - Val - (D)-Leu
- Glu - Gly - Gln - Ala - Ala - Lys - Glu - Phe - Ile - Ala - Trp - Leu - Val - Lys - Gly -
Gly - NH2

*Spectre de masse :* ESI - MS : m/z = 3138

### EXEMPLE 17 :

His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3178

### EXEMPLE 18 :

His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3201

### EXEMPLE 19 :

His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
NH2

*Spectre de masse :* ESI - MS : m/z = 3234

### EXEMPLE 20 :

His - Ala - Glu - Gly - Thr - Phe - Thr - Ser - Asp - Val - Ser - Ser - Tyr - Leu - Glu -
Gly - Gln - Ala - Ala - Lys - Glu - Ile - Ile - Ala - Trp - Leu - Val - Lys - Gly - Gly -
NH2

*Spectre de masse :* ESI - MS : m/z = 3162

### EXEMPLE 21 :

His - Ala - Glu - Gly - Thr - Phe - Thr - Ser - Asp - Val - Ser - Ser - Tyr - Leu - Glu -
Gly - Gln - Ala - Ala - Lys - Glu - Val - Ile - Ala - Trp - Leu - Val - Lys - Gly - Gly -
NH2

*Spectre de masse :* ESI - MS : m/z = 3146

### EXEMPLE 22 :

His - Ala - Glu - Gly - Thr - Phe - Thr - Ser - Asp - Val - Ser - Ser - Tyr - Leu - Glu -
Gly - Gln - Ala - Ala - Lys - Glu - His - Ile - Ala - Trp - Leu - Val - Lys - Gly - Gly -
NH2

*Spectre de masse :* ESI - MS : m/z = 3184

### EXEMPLE 23 :

His - Ala - Glu - Gly - Thr - Phe - Thr - Ser - Asp - Val - Ser - Ser - Tyr - Leu - Glu -
Gly - Gln - Ala - Ala - Lys - Glu - Asp - Ile - Ala - Trp - Leu - Val - Lys - Gly - Gly -
NH2

*Spectre de masse :* ESI - MS : m/z = 3162

### EXEMPLE 24:

His - Ala - Glu - Gly - Thr - Phe - Thr - Ser - Asp - Val - Ser - Ser - Tyr - Leu - Glu -
Gly - Gln - Ala - Ala - Lys - Glu - Phe - Leu - Ala - Trp - Leu - Val - Lys - Gly - Gly -
NH2

*Spectre de masse :* ESI - MS : m/z = 3194

### EXEMPLE 25 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Val - Ala - Trp - Leu - Val - Lys - Gly - Gly -
NH2

*Spectre de masse :* ESI - MS : m/z = 3180

### EXEMPLE 26 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Tyr - Ala - Trp - Leu - Val - Lys - Gly - Gly -
NH2

*Spectre de masse :* ESI - MS : m/z = 3245

### EXEMPLE 27 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Arg - Ala - Trp - Leu - Val - Lys - Gly - Gly -
NH2

*Spectre de masse :* ESI - MS : m/z = 3238

### EXEMPLE 28 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Glu - Ala - Trp - Leu - Val - Lys - Gly - Gly -
NH2

*Spectre de masse :* ESI - MS : m/z = 3210

### EXEMPLE 29 :

Phe - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2

*Spectre de masse :* ESI - MS : m/z = 3206

### EXEMPLE 30 :

His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg-
Gly - NH2

*Spectre de masse :* ESI - MS : m/z = 3255

### EXEMPLE 31 :

His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3354

### EXEMPLE 32 :

His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3334

### EXEMPLE 33 :

His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2

*Spectre de masse :* ESI - MS : m/z = 3312

### EXEMPLE 34 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2

*Spectre de masse :* ESI - MS : m/z = 3351

### EXEMPLE 35 :

His - (D)-Ala - Glu - Gly - Thr - Phe - Thr - Ser - Aib - Val - Ser - Ser - Tyr - Leu -
Glu - Gly - Gln - Ala - Ala - Lys - Glu - Phe - Ile - Ala - Trp - Leu - Val - Lys - Gly -
Gly - NH2

*Spectre de masse :* ESI - MS : m/z = 3316

### EXEMPLE 36 :

His - (D)-Ala - Glu - Gly - Thr - Phe - Thr - Ser - Asp - Val - Ser - Ser - Tyr - Leu -
Glu - Gly - Gln - Ala - Ala - Lys - Nva - Phe - Ile - Ala - Trp - Leu - Val - Lys - Gly -
Gly - NH2

*Spectre de masse :* ESI - MS : m/z = 3165

### EXEMPLE 37 :

His - (D)-Ala - Glu - Gly - Thr - Phe - Thr - Ser - Asp - Val - Ser - Ser - Tyr - Leu -
Glu - Gly - Gln - Ala - Ala - Lys - Nle - Phe - Ile - Ala - Trp - Leu - Val - Lys - Gly -
Gly - NH2

*Spectre de masse :* ESI - MS : m/z = 3180

### EXEMPLE 38 :

His - (D)-Ala - Glu - Gly - Thr - Phe - Thr - Ser - Asp - Val - Ser - Ser - Tyr - Leu -
Glu - Gly - Gln - Ala - Ala - Lys - Aib - Phe - Ile - Ala - Trp - Leu - Val - Lys - Gly -
Gly - NH2

*Spectre de masse :* ESI - MS : m/z = 3152

### EXEMPLE 39 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - (1)-Nal - Ile - Ala - Trp - Leu - Val - Lys - Gly
- Gly - NH2

*Spectre de masse :* ESI - MS : m/z = 3230

### EXEMPLE 40 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - (2)-Nal - Ile - Ala - Trp - Leu - Val - Lys - Gly
- Gly - NH2

*Spectre de masse :* ESI - MS : m/z = 3231

### EXEMPLE 41 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phg - Ile - Ala - Trp - Leu - Val - Lys - Gly -
Gly - NH2

*Spectre de masse :* ESI - MS : m/z = 3183

### EXEMPLE 42 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Nva - Ala - Trp - Leu - Val - Lys - Gly -
Gly - NH2

*Spectre de masse :* ESI - MS : m/z = 3180

### EXEMPLE 43 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Nle - Ala - Trp - Leu - Val - Lys - Gly -
Gly - NH2

*Spectre de masse :* ESI - MS : m/z = 3115

### EXEMPLE 44 :

His - (D)-Ala - Glu - Gly - Thr - Phe - Thr - Ser - Aib - Val - Ser - Ser - Tyr - Leu -
Glu - Gly - Gln - Ala - Ala - Lys - Aib - (1)-Nal - Nle - Ala - Trp - Leu - Val - Lys -
Gly - Gly - NH2

*Spectre de masse :* ESI - MS : m/z = 3158

En utilisant le procédé décrit dans l'exemple 1, en remplaçant la résine Fmoc-PAL-PEG-PS par une résine Moc-Gly-PAL-PEG-PS, les composés des exemples 45 à 49 sont obtenus.

### EXEMPLE 45 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - OH

*Spectre de masse :* ESI - MS : m/z = 3192

### EXEMPLE 46 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OH

*Spectre de masse :* ESI - MS : m/z = 3194

### EXEMPLE 47 :

His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OH

*Spectre de masse :* ESI - MS : m/z = 3180

### EXEMPLE 48 :

Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - OH

*Spectre de masse :* ESI - MS : m/z = 3206

### EXEMPLE 49 :

His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OH

*Spectre de masse :* ESI - MS : m/z = 3198

En utilisant le procédé décrit dans l'exemple 1, en remplaçant la résine Fmoc-PAL-PEG-PS par une résine Fmoc-Gly-PAL-PEG-PS, et en remplaçant le réactif K par un mélange triéthylamine/méthanol, les composés des exemples 50 à 54 sont obtenus.

### EXEMPLE 50 :

His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
OH

*Spectre de masse :* ESI - MS : m/z = 3210

### EXEMPLE 51 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - OCH3

*Spectre de masse :* ESI - MS : m/z = 3212

### EXEMPLE 52 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OCH3

*Spectre de masse :* ESI - MS : m/z = 3209

### EXEMPLE 53 :

His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OCH[?]

*Spectre de masse :* ESI - MS : m/z = 3197

### EXEMPLE 54 :

Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - OCH3

*Spectre de masse :* ESI - MS : m/z = 3220

En utilisant le procédé décrit dans l'exemple 1, en remplaçant la résine Fmoc-PAL-PEG-PS par une résine Fmoc-Gly-PAL-PEG-PS, et en remplaçant le réactif K par un mélange triéthylamine/isopropanol, les composés des exemples 55 à 57 sont obtenus.

### EXEMPLE 55 :

His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OCH(CH3)2

*Spectre de masse :* ESI - MS : m/z = 3240

### EXEMPLE 56 :

His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - OCH(CH3)2

*Spectre de masse :* ESI - MS : m/z = 3238

### EXEMPLE 57 :

His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
OCH(CH3)2

*Spectre de masse :* ESI - MS : m/z = 3237

### ETUDE PHARMACOLOGIQUE

### EXEMPLE A : Etude de liaison aux récepteurs du GLP-1

Les études de liaison aux récepteurs du GLP-1 ont été réalisées en utilisant des membranes RIN T3 dans un tampon 60 mM Tris-HCl pH 7.5, contenant 4 % de BSA et 750 µg/ml de bacitracin. Les membranes (20 à 30 µg) sont incubées dans un volume final de 500 µl avec environ 15 fmol de [125I]-GLP-1 (50 000 cpm) et le compétiteur froid pendant 45 min à 37°C. La réaction est arrêtée par addition de 750 µl de tampon KRP froid, pH 7.5 contenant 3 % de BSA. Le milieu est centrifugé à 12.000 × g (4°C, 5 min). Le culot est resuspendu dans ml de tampon KRP froid, sédimenté par une autre centrifugation et la radioactivité est mesurée Les résultats sont exprimés en IC₅₀.

### Résultats :

Les résultats obtenus révèlent pour les composés de l'invention une très haute affinité pour les récepteurs du GLP-1.
C'est le cas notamment du composé de l'exemple 7 qui possède une affinité de 3,3 10⁻¹⁰ M.

### EXEMPLE B : Détermination du caractère agoniste ou antagoniste

Le caractère agoniste des composés de l'invention a été déterminé en mesurant la production d'AMP cyclique après activation du récepteur par les différents produits à tester.

Des cellules RIN T3 sont cultivées pendant 6 jours et le milieu de culture est changé 1 jour avant l'expérience. Les cellules (3.10⁵ par puits) sont lavées deux fois avec du DMEM avant addition de 0,5 ml de DMEM contenant 1 % de BSA, 0,2 ml de IBMX et le peptide à tester. Après 20 minutes d'incubation à 25°C, l'AMPc intracellulaire est extrait, succinylé et quantifié par radioimmuno essai.

La valeur de référence à 100 % correspond à la production d'AMPc induite par une concentration de 10⁻⁸ M de GLP-1 et la valeur à 0 % correspond à la production basale en l'absence de GLP-1.

Les résultats sont exprimés en EC₅₀ qui est la concentration qui induit 50 % de la production d'AMPc obtenue par une concentration de 10⁻⁸ M de GLP-1.

### Résultats :

Les composés de l'invention présentent un caractère agoniste. Ils augmentent la production d'AMPc et ont des valeurs d'EC₅₀ nanomolaires ou subnanomolaires. A titre d'exemple, le composé de l'exemple 7 possède une EC₅₀ de 1,02 10⁻⁹ M.

### EXEMPLE C : Etude de la stimulation de la production d'insuline sur cellules en culture

La stimulation de la production d'insuline induite par les composés de l'invention est étudiée sur des cellules Min6 (5 × 10⁵ cellules par plaques dans 0 5 ml de milieu de culture). 18 heures avant l'expérience. le milieu de culture est pipetté et les celiules sont lavés deux fois avec du DRB pH 7.5 contenant 0,1 % de BSA. Les cellules sont alors préincubées pendant une heure dans du DRB-BSA contenant 1mM de glucose et ensuite incubées dans du DRB-BSA contenant différentes concentration de glucose et de compose à tester. Après incubation, le milieu est collecté, centrifugé pendant 5 minutes et stocké à -20°C. La sécrétion d'insuline est déterminée par radioimmuno essai en utilisant de l'insuline porcine marquée à l'iode 125 et l'anticorps antiinsuline de Kervran de cochon d'Inde.

### Résultats :

Les composés de l'invention sont capables de stimuler la sécrétion d'insuline de facon plus importante que le ^{t}GLP-1.

### EXEMPLE D : Etude de la stabilité métabolique

Chaque composé est dissout dans 50 mg/ml de BSA dans une solution aqueuse à 1 % d'acide trifluoroacétique, de façon à obtenir une concentration de 1 mg/ml.

Un aliquot de 50 µg/ml d'une solution contenant le peptide à étudier est incubée dans du plasma humain à 37°C Une méthode d'analyse en ligne par CLHP permet de mesurer la quantité de peptide au temps T0, puis après 5, 10, 15, 30 et 60 minutes d'incubation.

Des échantillons de 50 µl de la solution à 1 mg/ml du peptide préparée précédemment est ajoutée à 940 µl de TRIS 0,1M à pH=8,0 Après analyse de la solution par CLHP afin de doser la quantité de peptide, 10 µl d'une solution aqueuse contenant 48 miliunités de Dipeptidyl peptidase IV (DPP IV) sont ajoutés, et les échantillons sont analysées par CLHP au temps T0 et après 10 minutes d'incubation à 37°C.

Les résultats des mesures permettent d'évaluer la quantité de peptide restant et sont exprimés en pourcentage par rapport à T0.

### Résultats :

Il apparaît que les composés de l'invention possèdent une stabilité supérieure à celle du peptide naturel.
A titre d'exemple, les résultats obtenus avec le ^{t}GLP-1 et avec le composé de l'exemple 7 sont regroupés dans le tableau suivant.

| **Composé** | **Temps d'incubation** | **Plasma humain (% peptide restant)** | **DPP IV % peptide restant** |
|---|---|---|---|
| Exemple 7 | 5 | 100 | - |
| | 10 | 100 | 100 |
| | 15 | 100 | - |
| | 30 | 100 | - |
| | 60 | 100 | - |
| ^{t}GLP-1 | 5 | 83 | - |
| | 10 | 65 | 8 |
| | 15 | 53 | - |
| | 30 | 29 | - |
| | 60 | 10 | - |

### EXEMPLE E : Activité antihyperglycémiante

L'activité antihyperglycémiante des dérivés de l'invention a été recherchée sur des rats mâles normaux Wistar d'environ 250 g âgés de trois mois. L'homéostasie a été évaluée par un test de tolérance au glucose.

### . Test de tolérance au glucose par voie intraveineuse (IVGTT)

Le glucose est dissout dans une solution aqueuse de NaCI à 0,9 % et administré par la voie de la veine saphène à des rats anesthésiés au pentobarbital (60 mg kg⁻¹, *IP*). Les échantillons de sang sont collectés séquentiellement par les vaisseaux de la queue avant et 2, 5, 10, 15. 20 et 30 minutes après l'injection du glucose. Ils sont ensuite centrifugés et le plasma est separé. La concentration en glucose plasmatique est déterminée immédiatement sur un aliquot de 10 µl et le plasma restant est conservé à -20°C, jusqu'à la détermination de la concentration en insuline par radioimmuno essai,

Une injection unique en *iv* du produit à tester est effectuée à des rats à jeun anesthésiés au pentobarbital immédiatement après la charge en glucose selon le protocole décrit par Hendrick et al. (Metabolism, 1993, 42, 1),

### . Méthodes analytiques

La concentration en glucose plasmatique est déterminée par utilisation d'un analyseur de glucose (Beckman Inc., Fullerton, CA). La tolérance au glucose est mesurée par rapport à deux paramètres · ΔG et K.

ΔG représente l'augmentation de la glycémie au dessus de la ligne de base. intégrée sur une période de 30 minutes, après surcharge en glucose à la dose de 1 g/kg.
K est la vitesse de disparition du glucose entre 5 et 30 minutes, après administration du glucose.

Il apparaît que les composés de l'invention ont une activité insulinosecrétrice et antihyperglycémiante équivalente ou supérieure à celle du ^{t}GLP₁, présentent une durée d'action supérieure et une plus grande stabilité métabolique in vivo.

### EXEMPLE F : Composition pharmaceutique : soluté injectable

| | |
|---|---|
| Composé de l'exemple 7 | 10 mg |
| Eau distillee pour préparations injectables | 25 ml |

## Revendications

1. Composés peptidiques qui sont : 
•  His - Ala - Glu - Gly - Thr - Phe - Thr - Ser - Asp - Val - Ser - Ser - Tyr - (D)-Leu -
Glu - Gly - Gln - Ala - Ala - Lys - Glu - Phe - Ile - Ala - Trp - Leu - Val - Lys - Gly -
Arg - Gly - NH2
 
• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH₂
 
• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Gln - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH₂
 
• His - Ala - His - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2.
 
• His - Ala - (D)-Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Leu- Glu - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Gly - NH2
 
• His - Val - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• Afp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Ser - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Gly - NH2
 
•  His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - NH2
 
•  Phe - (D)-Ala - Gln - Gly - Thr - Phe - Thr - Ser - Asp - Val - Ser - Ser - Val - (D)-Leu -
Glu - Gly - Gln - Ala - Ala - Lys - Glu - Phe - Ile - Ala - Trp - Leu - Val - Lys - Gly -
Gly - NH2
 
• His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
NH2
 
•  His - Ala - Glu - Gly - Thr - Phe - Thr - Ser - Asp - Val - Ser - Ser - Tyr - Leu - Glu -
Gly - Gln - Ala - Ala - Lys - Glu - Ile - Ile - Ala - Trp - Leu - Val - Lys - Gly - Gly - NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Val - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - His - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Asp - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Leu - Ala - Trp - Leu - Val - Lys - Gly - Gly -
NH2
 
●  His - Ala - Glu - Gly - Thr - Phe - Thr - Ser - Asp - Val - Ser - Ser - Tyr - Leu - Glu -
Gly - Gln - Ala - Ala - Lys - Glu - Phe - Val - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Tyr - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Arg - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Phe - Glu - Ala - Trp - Leu - Val - Lys - Gly - Gly -
NH2
 
• Phe - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg-
Gly - NH2
 
• His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Ser - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Arg - Gly -
NH2
 
•  His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2.
 ainsi que leurs sels d'addition à un acide ou à une base pharmaceutiquement acceptable.

2. Procédé de préparation des composés peptidiques selon la revendication 1 **caractérisé en ce qu'**ils peuvent être obtenus par synthèse séquentielle sur phase solide à partir d'amino-acides protégés, par synthèse à partir de fragments peptidiques en solution, ou éventuellement par combinaison de ces deux techniques, dérivés des composés peptidiques selon la revendication 1 que l'on transforme, si nécessaire, en leurs sels d'addition à un acide ou à une base pharmaceutiquement acceptable.

3. Compositions pharmaceutiques contenant comme principe actif au moins un composé selon la revendication 1, seul ou en combinaison avec un ou plusieurs excipients ou véhicules inertes, non toxiques, pharmaceutiquement acceptables.

4. Compositions pharmaceutiques selon la revendication 3 contenant au moins un principe actif selon la revendication utiles comme agoniste du ^{t}(GLP-1) dans le traitement des pathologies dans lesquelles le ^{t}(GLP-1) intervient.

5. Composition pharmaceutique selon la revendication 4 contenant au moins un principe actif selon la revendication 1 utile dans le traitement du diabète de type II non insulinodépendant, de l'obésité, et dans le diabète de type I.

## Patentansprüche

1. Peptidverbindungen, nämlich: 
• His - Ala - Glu - Gly - Thr - Phe - Thr - Ser - Asp - Val - Ser - Ser - Tyr - (D)-Leu -
Glu - Gly - Gln - Ala - Ala - Lys - Glu - Phe - Ile - Ala - Trp - Leu - Val - Lys - Gly -
Arg - Gly - NH2
 
• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2
 
• Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Gln - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2.
 
• His - Ala - His - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2.
 
• His - Ala - (D)-Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Leu- Glu - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Gly - NH2
 
• His - Val - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• Afp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Ser - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Gly - NH2
 
• His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - NH2
 
Phe - (D)-Ala - Gln - Gly - Thr - Phe - Thr - Ser - Asp - Val - Ser - Ser - Val - (D)-Leu -
Glu - Gly - Gln - Ala - Ala - Lys - Glu - Phe - Ile - Ala - Trp - Leu - Val - Lys - Gly -
Gly - NH2
 
• His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Ile - Ile - Ala - Trp - Leu - Val - Lys - Gly - Gly - NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Val - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - His - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Asp - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Leu - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Val - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Tyr - Ala - Trp - Leu - Val - Lys - Gly - Gly -
NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Phe - Arg - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Glu - Ala - Trp - Leu - Val - Lys - Gly - Gly -
NH2
 
Phe - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg-
Gly - NH2
 
• His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
• His - Ala - Ser - Gly - Thr - Phe - Thr - Ser - Asp - Val - Ser - Ser - Tyr - Leu - Glu - Gly
- Gln - Ala - Ala - Lys - Glu - Phe - Ile - Ala - Trp - Leu - Val - Lys - Gly - Arg - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2.
 sowie deren Additionssalze mit einer pharmazeutisch annehmbaren Säure oder Base.

2. Verfahren zur Herstellung der Peptidverbindungen nach Anspruch 1, **dadurch gekennzeichnet, daß** sie erhältlich sind durch sequentielle Synthese auf fester Phase ausgehend von geschützten Aminosäuren, durch Synthese ausgehend von Peptidfragmenten in Lösung oder gegebenenfalls durch Kombination dieser beiden Methoden,
welche Peptidverbindungen nach Anspruch 1 man erforderlichenfalls in ihre Additionssalze mit einer pharmazeutisch annehmbaren Säure oder Base umwandelt.

3. Pharmazeutische Zubereitungen enthaltend als Wirkstoff mindestens eine Verbindung nach Anspruch 1, allein oder in Kombination mit einem oder mehreren inerten, nicht-toxischen, pharmazeutisch annehmbaren Trägermaterialien oder Bindemitteln.

4. Pharmazeutische Zubereitungen nach Anspruch 3, enthaltend mindestens einen Wirkstoff nach Anspruch 1 zur Verwendung als Agonist von ^{t}(GLP-1) bei der Behandlung von Erkrankungen, bei denen ^{t}(GLP-1) eine Rolle spielt.

5. Pharmazeutische Zubereitungen nach Anspruch 4, enthaltend mindestens einen Wirkstoff nach Anspruch 1 für die Behandlung des nicht-insulinabhängigen Diabetes Typ II, der Fettsucht und des Diabetes Typ I.

## Claims

1. Peptide compounds which are 
•	His - Ala - Glu - Gly - Thr - Phe - Thr - Ser - Asp - Val - Ser - Ser - Tyr - (D)-Leu -
Glu - Gly - Gln - Ala - Ala - Lys - Glu - Phe - Ile - Ala - Trp - Leu - Val - Lys - Gly -
Arg - Gly - NH2
 
•	Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2
 
•	Trp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
•	His - Ala - Gln - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2
 
His - Ala - His - Gly - Thr - Phe - Thr - Ser - Asp - Val - Ser - Ser - Val - Leu - Glu -
Gly - Gln - Ala - Ala - Lys - Glu - Phe - Ile - Ala - Trp - Leu - Val - Lys - Gly - Arg -
Gly - NH2.
 
His - Ala - (D)-Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
•	His - (D)-Ala - Glu - Gly - Thr - Phe - Thr - Ser - Asp - Val - Ser - Ser - Tyr - Leu -
Glu - Gly - Gln - Ala - Ala - Lys - Glu - Phe - Ile - Ala - Trp - Leu - Val - Lys - Gly -
Gly - NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
His - Leu- Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Val - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	Afp - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
His - Ala - Asp - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
• His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	Phe - (D)-Ala - Glu - Gly - Thr - Phe - Thr - Ser - Asp - Val - Ser - Lys - Tyr - Leu -
Glu - Gly - Gln - Ala - Val - Lys - Glu - Phe - Ile - Ala - Trp - Leu - Val - Lys - Gly -
Gly - NH2
 
•	Phe - (D)-Ala - Gln - Gly - Thr - Phe - Thr - Ser - Asp - Val - Ser - Ser - Val - (D)-Leu
- Glu - Gly - Gln - Ala - Ala - Lys - Glu - Phe - Ile - Ala - Trp - Leu - Val - Lys - Gly -
Gly - NH2
 
•	His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Trp - Ile - Ala - Trp - Leu - Val - Lys - Gly - Gly -
NH2
 
• His - Ala - Glu - Gly - Thr - Phe - Thr - Ser - Asp - Val - Ser - Ser - Tyr - Leu - Glu -
Gly - Gln - Ala - Ala - Lys - Glu - Ile - Ile - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Val - Ile - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - His - Ile - Ala - Trp - Leu - Val - Lys - Gly - Gly -
NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Asp - Ile - Ala - Trp - Leu - Val - Lys - Gly - Gly -
NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Phe - Leu - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Phe - Val - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Phe - Tyr - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Phe - Arg - Ala - Trp - Leu - Val - Lys - Gly - Gly -
NH2
 
•	His - Ala - Glu - Gly - Thr - Phe - Thr - Ser - Asp - Val - Ser - Ser - Tyr - Leu - Glu -
Gly - Gln - Ala - Ala - Lys - Glu - Phe - Glu - Ala - Trp - Leu - Val - Lys - Gly - Gly -
NH2
 
•	Phe - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Gly -
NH2
 
•	His - Ala - Lys - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg-
Gly - NH2
 
His - Ala - Met - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
•	His - Ala - Leu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
His - Ala - Ser - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - Leu - Val - Lys - Gly - Arg -
Gly - NH2
 
His - Ala - Glu - Gly - Thr - Phe - Thr - Ser - Asp - Val - Ser - Ser - Tyr - Leu - Glu -
Gly - Gln - Ala - Ala - Lys - Glu - Phe - Ile - Ala - Trp - (D)-Leu - Val - Lys - Gly -
Arg - Gly - NH2
 and addition salts thereof with a pharmaceutically acceptable acid or base.

2. Process for the preparation of peptide compounds according to claim 1, **characterised in that** they may be obtained by solid phase sequential synthesis starting from protected amino acids, by synthesis starting from peptide fragments in solution, or optionally by a combination of those two techniques,
which peptide compounds according to claim 1 are converted, if necessary, into their addition salts with a pharmaceutically acceptable acid or base.

3. Pharmaceutical compositions comprising as active ingredient at least one compound according to claim 1, on its own or in combination with one or more pharmaceutically acceptable, inert, non-toxic excipients or carriers.

4. Pharmaceutical compositions according to claim 3 comprising at least one active ingredient according to claim 1 for use as an agonist of ^{t}(GLP-1) in the treatment of pathologies in which ^{t}(GLP-1) is involved.

5. Pharmaceutical composition according to claim 4 comprising at least one active ingredient according to claim I for use in the treatment of non-insulin-dependent type II diabetes, obesity, and in type I diabetes.
